# EUROPEAN PATENT APPLICATION

(11) **EP 2 420 185 A2**
(43) Date of publication of application: **22.02.2012**
(21) Application number: 11178307.2
(22) Date of filing: 13.04.2006
(51) Int. Cl.: A61B 5/00

(54) **Apparatus and system for monitoring**

(30) Priority: 14.04.2005 GB 0507486; 14.04.2005 GB 0507485
(62) Divisional of application: 06726754.2
(71) Applicant: Hidalgo Limited, Swavesay Cambridgeshire CB24 4UQ (GB)
(72) Inventor: Pisani, Justin, Swavesey, Cambridgeshire CB24 4UQ (GB); Howard, Peter, Swavesey, Cambridgeshire CB24 4UQ (GB); Cade, Daniel, Swavesey, Cambridgeshire CB24 4UQ (GB); Ward, Stephen, Swavesey, Cambridgeshire CB24 4UQ (GB)
(74) Representative: Widdowson, Nicholas Edward

(57) **Abstract**

A monitoring device wearable by a person to be monitored, comprising: one or more sensing means for sensing cardio, respiratory, physiological and/or other information from the person; processing means for analysing the sensed information; memory means for storing the sensed and/or analysed information; and communication means for transmitting at least the analysed information. At least one waveform acquired from the sensed cardio, respiratory, physiological and/or other information is digitised in real-time; analysis of the sensed and/or digitised information is performed in real-time and a welfare indication of the person computed in real-time; and the computed welfare indication of the person is transmitted by the communication means and/or stored in the memory means.

## Description

The invention relates to monitoring devices. In particular, the invention relates to a monitoring device wearable by a person to be monitored. Further, the invention relates to a monitoring device wearable by an ambulatory person to be monitored. The invention also concerns a monitoring system for real-time monitoring of one or more ambulatory persons.

A sensing device may be useful, for example, in the monitoring of individuals who are undertaking activities, or who are placed in environments, where an increased risk of injury or physical trauma may exist, and where continuous medical supervision from a health care practitioner may not be possible. Environments and activities which present such increased risks to a user may include, for example, zones of military operations, hazardous plants, public safety enforcement and lone-working individuals.

Typically, the sensing devices will sense a person's physiological information, in order to provide an indication of the physical welfare of that person. Additionally, a monitoring station may receive the physiological information and personnel at the monitoring station may use this data to assist in the determination of well-being of the user and to assist in determining the need for appropriate interventions, such as, despatching medical expertise to the person.

Typically, the types of physiological information (signals) to be sensed may include, for example, the user's electrocardiogram (ECG), breathing effort rate, skin temperature, blood oxygenation level, pulsatile waveform, body orientation, body motion, and/or body gravitational force loading.

Devices which extract, process and display one or more of the above signals from users in real-time are known in the art but are generally intended for the monitoring of a single individual with known or suspected ailments. The operation of the sensing device is typically under the direct control of a healthcare practitioner who will normally be co-located to, and within visual contact of, the person being monitored - which situation is exemplified by a patient in a hospital.

Analysis and visual display of the data is undertaken, usually, after collection of the complete data signals from the person, which signals are transferred to a separate, non-wearable unit, which processes and displays the data. Such units are typically dedicated to the person being monitored. It will also be appreciated that these units may be transportable, by trolleys for example, but they are not wearable, in the sense that a person would not be normally mobile while wearing the device. In some instances an extra intermediate unit is carried by, or placed near to, the user to condition and relay the signals to the processing and display device. For users with known or suspected ailments, the transfer and monitoring of these signals by a healthcare practitioner is appropriate, but for the asymptomatic user they present an unnecessary and restrictive overhead.

Devices also exist, and are known in the art, which are targeted at ambulatory users, for example, in order to undertake the monitoring of a users ECG over a longer period whilst they undertake normal day to day activities. Typically such devices allow the user a limited degree of ambulation but are not designed for completely unrestricted physical activity by the user. In addition such devices rely on the recording of the raw physiological data signals on a storage media and then the transfer of this data to a monitoring station which processes and analyses the data in retrospect. These devises are not capable of real-time analysis of the data signals and, hence, the assessment of the signals recorded is done after the completion of monitoring, and after transferring the data signals.

Accordingly, it is clear that prior art devises exist for monitoring some physiological information from a person. However, such devises are not intended for use with ambulatory people who might carry out a range of activities, of varying physical intensity. It can be seen that the characteristics of both types of prior art device show that they are not suitable for remote monitoring of single or groups of potentially geographically diverse individuals without specific ailments or suspected conditions and who need to be freely capable of undertake their day to day activities, irrespective of the physical intensity of the activity, without excess restriction being placed on the person by the sensor device itself.

An object of the invention is to provide a monitoring device which will not restrict the movement of an ambulatory person, irrespective of the physical intensity of the activity, and which provides an indication of the physical welfare of the person. In particular, the device would be intended for general use by an active individual, without offering significant restriction to the wearer, in terms of what activities they may undertake, the clothing they may wear and the duration they may use the device.

Accordingly, in a first aspect the invention provides a monitoring device wearable by a person to be monitored, comprising:
one or more sensing means for sensing cardio, respiratory, physiological and/or other information from the person;
processing means for analysing the sensed information;
memory means for storing the sensed and/or analysed information; and
communication means for transmitting at least the analysed information, wherein:
   at least one waveform acquired from the sensed cardio, respiratory, physiological and/or other information is digitised in real-time;
   analysis of the sensed and/or digitised information is performed in real-time and a welfare indication of the person computed in real-time; and
   the computed welfare indication of the person is transmitted by the communication means and/or stored in the memory means.

Preferably, the communication means is capable of transmitting sensed information. Most preferably, the communication means is capable of transmitting digitised information.

The monitoring device is capable of transmitting the welfare indication, the sensed information and/or the digitised information in real-time. In particular, this can be every 15 seconds, although this duration can be altered to meet specific needs.

Preferably, the communication means may be part of a radio and/or satellite communications network.

Most preferably, all waveforms acquired from the sensed cardio, respiratory, physiological and/or other information may be digitised in real-time.

Further preferably, all information is digitised in real-time.

The sensing means may be one or more skin electrodes. In particular, the sensing means may be one or more electrodes and associated electronics circuitry. Additionally, the one or more sensing means may comprise at least two sensing means.

Preferably, at least part of the memory is a buffer-type memory.

The processor is capable of processing at least two forms of information selected from cardio, respiratory, physiological and/or other information, to derive data relating to a welfare indication of a wearer. Further, the processor is capable of processing the (primary) cardio, respiratory, physiological and/or other information to derive secondary cardio, respiratory, physiological and/or other information and the processor is capable of processing at least two forms of information selected from the primary and/or secondary cardio, respiratory, physiological and/or other information to derive data relating to a welfare indication.

Preferably, the monitoring device comprises a plurality of integrated sensors for detecting the cardio, respiratory, physiological and/or other information.

The welfare indication may be determinable by analysis and/or comparison of newly received cardio, respiratory, physiological and/or other information with thresholds from configurable data stored in the memory.

Advantageously, the monitoring device is capable of detecting cardio, respiratory, physiological and/or other information relating to one or more of the following:
a) an electrical view of the heart of a person;
b) the respiration effort of a person;
c) the blood oxygen level of a person;
d) the skin surface impedance of a person;
e) whether there is correct skin electrode and person contact;
f) the skin surface temperature of a person;
g) whether a specific activity is being undertaken by a person;
h) whether a person has been effected by an impact;
i) the body orientation of a person;
j) the movement of a person;
k) the level of ambulation of a person;
l) the absence of expected data;
m) the cognitive state of a person;
n) a person's own assessment of welfare; and/or
o) whether excessive gravitational forces are being exerted on a person.

Thresholds and configurable data may be modifiable for a specific person. Also, the thresholds and configurable data may be modifiable for a type of range of activities or environments. Further, the thresholds and configurable data may be modifiable as a result of contextual information relating to a person.

The configurable data is derivable from previous analysis and/or comparison of cardio, respiratory, physiological and/or other information and the thresholds. Further, the monitoring device may be capable of providing the configurable data from analysis of time-thresholds which conditions must be measured before a transition in the welfare indication occurs for one or more of the following conditions:
a) high, low or intermediate signal rates;
b) an absence of measurable signal rates;
c) the rate of change of an averaged signal rate;
d) averages of a measured signal rate;
e) the short-term average of a measured signal rate;
f) the long-term average of a measured data signal rate;
e) the normal or abnormal characteristics of a waveform; or
f) intermediate average of a measured signal rate;
g) the time-threshold periods for transitions and/or average windows.

Advantageously, the welfare indication may be capable of being overridden or reduced in severity by additional contextual information experienced by a person.

Contextual information may relate to one or more of the following:
a) whether a person is moving;
b) whether a person has been effected by an impact;
c) whether a person is carrying out a specific activity;
d) the current or recent level of ambulation of a person;
e) environmental factors experienced a person; or
f) the cognitive state of a person.

Environmental factors may include:
a) ambient temperature;
b) ambient pressure;
c) altitude;
d) humidity; or
e) relative motion of the person.

Preferably, the sensitivity of detection may be modifiable in response to the activity status, level of ambulation and/or body position detected by the monitoring device, and/or contextual information experienced by a person.

Most preferably, the monitoring device may be capable of sensing more than one measurement of cardio information. In particular, the monitoring device may be capable of detecting information relating to two measurements of heart rate. The measurements are provided by analysis of a person's ECG waveform, and/or a second alternative view of a person's ECG waveform and/or pulse train, using R-wave analysis or analysis of a person's blood oxygen pulsatile waveform.

Most preferably, the monitoring device may be capable of detecting more than one measurement of respiratory information. In particular, the monitoring device may be capable of detecting information relating to three measurements of respiration rate. The measurements are provided by chest expansion measurements, thoracic impedance pleythismography measurements and from measurements of electrocardiograph data.

The monitoring device may compare the more than one measurement of cardio information to provide a cardio confidence score. The monitoring device may compare the more than measurement of respiratory information to provide a respiratory confidence score.

Preferably, the monitoring device may analyse the cardio confidence score and the respiratory confidence score, together with data relating to the individual signal quality or contextual information to provide an overall confidence score.

The welfare indication may be selectable from: normal; low priority alert; high priority alert; and unknown/un-operative. Additionally, the welfare indication may comprise an additional state of absence of vital signs.

The monitoring device may be capable of modifying the severity of its welfare indication and the time threshold for indicating the welfare indication following detection of the absence, or substantial absence, of one or more cardio or respiratory measures.

In the situation that a person has, initially, a normal welfare indication, a second cardio and/or respiratory measurement is triggerable automatically following determination of an abnormal welfare indication.

In the situation that a person has, initially, a low-level abnormal welfare indication, a second cardio and/or respiratory measurement is triggerable automatically following determination of a progressively abnormal welfare indication.

The welfare indication provides the monitoring station with grades of normality (normal) and abnormality (inactive/in-operable or absence of vital signs) of the health (welfare) of a person.

Preferably, a secondary welfare indication may be provided by analysis of thermal and/or neurological information. As such, the cognitive state of a person may be manually determinable by the monitoring station requesting the wearer to carry out an action.

Alternatively, and/or additionally the cognitive state of a wearer may be automatically determinable following: a variable or set time period; an abnormal welfare indication; or evidence of excessive g-shock to a person, by the person being automatically requested to carry out an action. As such, the person may be requested by visual, audible, vibrational or other sensory means. The frequency of request can be varied depending upon the detection of a response or the type of response of a person. Further, an abnormal welfare indication may be cancellable or movable towards normal by a person responding to the request to carry out the action. The action of the person may be capable of modifying the welfare indication to indicate a worsening of the his/her welfare, or that assistance is required.

The monitoring device may be capable of providing a secondary welfare indication by analysis of a measure of physiological strain derivable from a function of heart rate of the person and the insulated skin temperature, and configurable data stored in the memory.

Most preferably, the monitoring device is capable of abbreviated disclosure, when only a subset of the digitised information is communicated to the monitoring station, or full-disclosure, when all digitised information is communicated to the monitoring station. Under full-disclosure, some or all of the waveforms of the cardio, respiratory, physiological and/or other information may be transmitted to the monitoring station. Full-disclosure may be activated automatically by determination of an abnormal welfare indication. It may be also manually-activatable by a person or by the monitoring station. The subset comprises, preferably, one or more of:
a) primary and/or secondary welfare indication;
b) heart and/or respiration rate;
c) skin temperature;
d) motion and/or activity level;
e) body orientation;
f) user identification information;
g) unit identification information;
h) unit self-check diagnostics; and/or
i) confidence scores.

Preferably, the monitoring device further comprises a request and response device, wearable by a person, for communication with the monitoring device or the monitoring station. In particular, the request and response device may be wrist-worn. It may also comprise one or more sensors and/or a watch. The sensors may be a heart rate sensor and/or an accelerometer.

Most preferably, the monitoring device may be capable of transmitting the welfare indication, the digitised cardio, respiratory, physiological and/or other information or the waveforms of the cardio, respiratory, physiological and/or other information direct to a monitoring station, or via intermediate transfer or monitoring equipment.

Advantageously, assessment of a person's welfare is optimised by transmittal and storage of wearer-personalisation information, environment information and/or activity information by the monitoring station, any intermediate equipment and/or the monitoring device.

In particular, two-way communication means may be provided between the monitoring device and a monitoring station or intermediate equipment. As such, the monitoring device may comprise a wireless transmitter and receiver for communication with the monitoring station or intermediate equipment. In addition or alternatively, communication between the monitoring device and the monitoring station or intermediate equipment may be provided by a wired connection.

The invention may comprise connectable external sensors for detection of further cardio, respiratory, physiological and/or other information. The external sensors may communicate by wired or wire-less connections.

Advantageously, the monitoring device may be capable of detecting the presence of motion of a person and using the evidence of motion to reduce the bandwidth of the cardio signal receiver to improve the signal to noise ratio and improve performance.

Advantageously, the monitoring device may be capable of detecting the presence of motion and body position of a person and using evidence of motion and body position to modify the signal gain, bandwidth and sensitivity of the respiratory signal receiver to improve performance.

Additionally, the monitoring station may be capable of uploading to the monitoring device contextual information and/or configurable information.

In a second aspect, the invention provides a monitoring device wearable by a person to be monitored, comprising:
a detachable anatomically-shaped sensor electronics module comprising processing means, memory means and communications means; and
a connector harness and/or other support wearable by a person, capable of attaching, or holding in sensing proximity, the sensor electronics module to a person, and comprising one or more sensing means, wherein the monitoring device:
   senses cardio, respiratory, physiological and/or other information from a person; and
   performs real-time analysis of the sensed information and computes a real-time welfare indication of the person for onwards transmission / communication.

Preferably, the one or more sensing means may be arranged to provide electrical/electronic communication with an attached sensor electronics module. Further, the one or more sensing means may comprise at least two sensing means. Most preferably, the sensing means is one or more skin electrodes. In particular, the sensing means is one or more skin electrodes and associated electronics circuitry.

Preferably, the communication means is part of a radio and/or satellite communications network.

The monitoring device may comprise means for detecting skin temperature, such as a thermistor.

Preferably, the monitoring device comprises means for detection of motion, body position and/or impact, such as, an accelerometer.

Preferably, the monitoring device further comprises a chest-expansion sensor, for example, a variable strain sensor. The chest expansion sensor may be provided as part of a yolk.

Preferably, the monitoring device comprises means for detecting blood oxygen levels of a user, for example, a reflectance-type sensor for pulse oximetry analysis.

Advantageously, the sensor electronics module is capable of real-time analysis of information to provide a welfare indication of a person. The sensor electronics module may be capable of acquiring, storing and digitising the waveform of the cardio, respiratory, physiological and/or other information for internal analysis and/or onwards transmission. Onwards transmission may be to a monitoring station or to intermediate equipment, such as, a further transmission device or a portable computer.

The sensor electronics module is capable of communication by wired or wireless means.

Preferably, the sensor electronics module is capable of measuring, processing, analysing and/or onwards transmission of information relating to one or more of the following:
a) an electrical view of the heart of a person;
b) the respiration effort of a person;
c) the blood oxygen level of a person;
d) the skin surface impedance of a person;
e) whether there is correct skin electrode and person contact;
f) the skin surface temperature of a person;
g) whether a specific activity is being undertaken by a person;
h) whether a person has been effected by an impact;
i) the body orientation of a person;
j) the movement of a person;
k) the level of ambulation of a person;
l) the absence of expected data;
m) the cognitive state of a person;
n) a person's own assessment of welfare; and/or
o) whether excessive gravitational forces are being exerted on a person.

Most preferably, the sensor electronics module is capable of measuring, processing, analysing and/or onwards transmission of more than one measurement of cardio information, for example, two distinct views of a person's electrocardiogram.

Most preferably, the sensor electronics module is capable of measuring, processing, analysing and/or onwards transmission of more than one measurement of respiratory information, for example, chest expansion measurements, skin impedance measurements and measurements from electrocardiograph data.

Particularly advantageously, the sensor electronics module is anatomically-shaped to fit the thoracic region of a person. As such, it may be shaped to fit in the region of the sternum and upper abdomen of a person. Further, it may comprise three lobes in a triangular configuration.

Preferably the wearable monitoring device comprises three skin electrodes. Skin electrodes may be spaced as far apart as possible, in the context of a person's body size. Preferably, the spacing is from 5cm to 15cm apart. Most preferably 10cm apart.

The connector harness and/or other support comprises one or more of the following:
a) an adhesive pad;
b) a yolk;
c) an item of clothing; or
d) standard electrocardiograph adhesive skin electrodes.

The adhesive pad may be anatomically-shaped to fit the thoracic region of a wearer, and/or it may be shaped to fit in the region of the sternum and upper abdomen of a wearer. As such, the adhesive pad may comprise three lobes in a triangular configuration.

Preferably, the yolk comprises an adjustable band capable of being located around the thorax of a wearer. It may also comprise an over the shoulder strap for preventing movement of the yolk.

Preferably, the item of clothing is a tight-fitting vest or T-shirt.

The sensor electronics module may be connectable to the connector harness and/or other support by conductive snap-rivet fittings. Preferably, at least three snap-rivet fittings are utilised.

Additionally, the sensor electronic module may comprise an electrical interconnect which enables connection of one or more of the following:
wired computing terminals;
auxiliary sensors;
an auxiliary pulse oximetry module;
a power source.

The electrical interconnect may be in the form of a data link for connection of auxiliary sensors, monitoring equipment, transmission equipment or any auxiliary electrical equipment.

The monitoring device may further comprise auxiliary, connectable sensor equipment, such as, a reflectance-type sensor for pulse oximetry analysis and/or a request and response device.

Preferably, the request and response device may alert a wearer. A person may communicate with the sensor electronics module or monitoring station using the request and response device. In particular, the device may be wrist-worn.

Onwards transmission of information from the sensor electronics module may be provided by wired or wireless means and the sensor electronics module may comprise a two-way transmitter for communication with a monitoring station or intermediate equipment.

Most preferably, a person to be monitored is an ambulatory person.

In a third aspect of the present in action; there is provided a monitoring system for monitoring of one or more persons comprising:
a monitoring device as claimed in any one of claims 1 to 69 or as claimed in any one of claims 70 to 122, worn by the or each person being monitored; and
one or more monitoring stations, wherein:
   the or each monitoring device is in communication with the one or more monitoring stations; and
   the one or more monitoring stations receive and monitor the computed welfare indication from the or each monitoring device to assess the wellbeing of each person being monitored.

The system is, preferably, capable of abbreviated disclosure, when only a subset of digitised information may be communicated to the monitoring station, or full-disclosure, when all digitised information may be communicated to the monitoring station. Under full-disclosure, some or all of the waveforms of the cardio, respiratory, physiological and/or other information may be transmitted to the monitoring station. Full-disclosure may be activated automatically by determination of an abnormal welfare indication. Alternatively, and additionally, full-disclosure may be manually activatable by a wearer or by the monitoring station.

The welfare indication is, preferably, selectable from: normal; low priority alert; high priority alert; and unknown/un-operative, but may also comprise an additional state of absence of vital signs.

The monitoring system may be capable of transmitting the welfare indication, the digitised cardio, respiratory, physiological and/or other information or the waveforms of the cardio, respiratory, physiological and/or other information to a monitoring station via intermediate transfer or monitoring equipment.

The monitoring station and monitoring device preferably communicate in a two-way manner by wired or wireless means.

In particular, configurable parameters may be determined, and adjusted, recorded and stored within the monitoring device whilst in a 'training mode', for use when the monitoring device is not in a training mode.

The invention provides a compact monitoring device worn by a user comprising:
a plurality of integrated sensors to record cardio respiratory physiological information from the user in real time;
a processing element which:
   processes the physiological information within the device to derive additional secondary physiological information such a rates, periodicity and signal quality; and
   processes two or more of the physiological or secondary physiological information items in real-time in order to derive a welfare indication of at least four levels (normal, low priority alert, high priority alert and unknown/inoperative); and
a transceiver device capable of communicating the welfare indication wirelessly to a mobile communications device periodically which can then forward this information to a remote (to the user) monitoring station for review and assessment.

The cardio respiratory welfare indication can be derived from configurable settings held within the device for one or more of the following conditions:
- high, low and intermediate signal rates;
- absence of measurable signal rates;
- rate of change of a averaged signal rate;
- long term average(s) of the measured signal rate;
- short-term average(s) of the measured signal rate; or
- the time thresholds with which these conditions must be measured before a transition occurs in the welfare indication;
for both individual or combinations of physiological information measured from the user.

The monitoring device can differentiate its welfare indication severity and also time to indication depending on whether the absence of one or more of cardio respiratory measures has been identified.

The welfare indication comprises of an additional state indicating a sustained absence of vital signs for a defined time period.

The accuracy and efficacy of the welfare indication can be improved by the generation of a confidence measure for some or all of the cardio respiratory signals monitored, by the inclusion of a secondary or higher fidelity means to measure the same physiological function within the sensor. Deduction of an overall confidence level, as a mathematical function of the individual signal quality and the comparison error between the two measures, may be formulated.

The secondary measurement may be enabled and disabled according to the welfare values provided, by making a subset of the total possible measurements of the users physiology. This minimises electrical power consumption and increases the spare processing capacity of the device. Additional secondary measurements may be provided by other body worn sensors which communicate by wired or wireless means to the device.

The cardio respiratory welfare indication setting can be overridden or reduced in severity by additional contextual information measured by the sensor including activity and ambulation level, in order to reject implausible combinations of cardio respiratory and contextual information and hence reduce the rate of false alarms.

A wearer's/user's neurological state may be measured by alerting the wearer by visual, audible or other sensory means and requiring the wearer to undertake a response action such as pressing a button or striking the sensor housing. A secondary means of welfare indication may be provided to independently indicate a user's basic cognitive ability by the result of a neurological response test triggered either by time, abnormal cardio respiratory indication, or evidence of excessive g-shock to the user body.

The alerting and response device may be a remote wrist worn device, a means to indicate the time and date to the user and optionally contains other sensing devices. The user's response may include a different action to indicate whether assistance is or is not required. In particular, the wrist-worn device may be a modified wrist watch.

In particular, a neurological response test may be initiated by the detection of an abnormal cardio respiratory indication by the sensor. The cardio respiratory welfare indication setting can be overridden or reduced in severity by the users response to a neurological response test and, hence, this may reduce the rate of false alarms. The frequency of neurological state measurement may be varied by the sensor depending on the detection and type of response from a user.

A measure of physiological strain can be derived from a mathematical function of the user's heart rate and insulated skin temperature, measured by the device. The results of which are used to provide a secondary means of welfare indication depending on the physiological strain value computed and the configure value (from configurable settings) within the sensor.

In particular, some or all of the physiological signals waveforms may also be transferred on request from the monitoring station. Some or all of the physiological signals waveforms may also be transferred automatically on processing of a welfare indication of type other than normal or by wearers request.

Additional interim values of the welfare indication can be derived to provide an indicator of increasing importance between the normal and abnormal conditions.

Assessment of a user's welfare is optimised by the sending and storage of user physiological personalisation information to the sensor.

Communication to the mobile communications device can be achieved via a wired connection.

The invention also provides a compact body-worn monitoring device, intended for use by a ambulatory user which enables the measurement, processing, analysis and onward transmission of multiple physiological parameters where said device comprises of:
an anatomically-shaped self-powered sensor electronics module unit which processes analyses and transfers the physiological information to remote device for capture, display, analysis or further processing either by wired or wireless means;
a single body worn connection assembly containing three or more integrated skin electrodes, which supports and
locates the sensor electronics module;
wherein the sensor electronics module is capable of measuring and processing:
- two or more distinct views of a user's electrocardiogram (ECG);
- the respiration effort of a user, by measuring electrical impedance or motion changes;
- the skin surface temperature;
- the body gravitational load in vertical and horizontal axes; and/or
- the skin surface electrical impedance.

The sensor electronics module is anatomically-shaped to fit the users thoracic cavity in an approximately triangular, three-lobed arrangement and shaped to fit between the sternum and abdomen.

The sensor connection assembly comprises a central connection conformal material piece shaped to fit between a user's sternum and abdomen in an approximate triangular, three-lobed configuration, where the central connection piece contains the means to connect and support the sensor electronics module (SEM) by three or more electrically conductive snap rivet fittings. The fittings connect to three or more body contacting conductive electrodes and sensors. The SEM is retained in place by a horizontal flexible fabric strap and one or two vertical fabric straps extending from the sternum point over the shoulder and reconnecting to the horizontal strap where the two meet at a user's back.

The SEM may contain an additional high density electrical interconnect which may be, optionally, connected via a male electrically conductive contact of similar dimensions and enables the connection of:
- wired computing terminals;
- an external pulse oximetery module held within the sensor connection unit;
- other sensors; or
- a power source to assist the power cells contained within the Sensor Electronics Module.

The horizontal strap also contains means to measure the user's breathing related chest movement by the incorporation of a variable impedance strain sensor, which connects to the sensor electronics module via conductive snap fittings or by the interconnect defined above.

The strap contain adjusters to allow the user to tension the sensor connection unit to the body optimally.

The sensor connection unit is produced in sizes to allow fitment to a broader range of body sizes.

The sensor connection assembly may be substituted by a conductive adhesive patch laminate structure of three electrodes conforming to the same triangular electrode configuration, where the adhesive patch uses the same connection fitting types and locations to allow it to connect to the same sensor electronics module without need for modification.

The sensor connection assembly consists of a fabric vest structure containing three or more electrodes conforming to the same triangular electrode configuration. The vest uses the same connection fitting types and locations to allow it to connect to the same sensor electronics module without need for modification.

The means to measure the users breathing related chest movement is provided by the incorporation of a horizontal variable impedance strain sensor which connects to the sensor electronics module via conductive snap fittings or by the interconnect defined above.

The sensor connection unit comprises a contact plate to connect electrically to the sensor electronics module and offers three or more individual electrode wires which may be used to connect to individual electrodes at other locations on the body and also to a separate pulse oximeter module.

The sensor electronics device is provided for measuring a users ECG. The sensor detects the presence of motion by measuring gravitational load variation on the body using an accelerometer and uses evidence of motion to reduce the bandwidth of the ECG signal receiver and, thus, improves the signal to noise of the ECG.

The sensor electronics device is provided for measuring a users breathing. The sensor detects the presence of motion and body position, by measuring gravitational load variation on the body using an accelerometer, and uses evidence of motion and body position to modify the signal gain, bandwidth and sensitivity of the breathing signal receiver and detector, and, thus, optimises the performance of the breathing detector.

The present invention also provides a monitoring device wearable by a user comprising:
a plurality of sensors to record physiological information from the user in real time;
a processing element which:
   processes the physiological information to derive additional secondary physiological information such a rates and periodicity;
   processes two or more of the physiological or secondary physiological information items in real time in order to derive welfare indication of a least two levels (abnormal/normal);
a transceiver device capable of communicating the welfare indication wirelessly to a mobile communications device periodically which can then forward this information to a remote (to the user) monitoring station for review and assessment.

The welfare indication comprises three states: red, amber and green.

Some or all of the physiological signals waveforms may also be transferred on request from the monitoring station.

Further, some or all of the physiological signals waveforms may also be transferred automatically on processing of a welfare indication of type abnormal or by wearer's request.

Interim values of the welfare assessment may be derived to provide an indicator of increasing importance between the normal and abnormal conditions.

The assessment of the users welfare is optimised by the sending of user personalisation information to the sensor.

Communication to the mobile communication device may be achieved via a wired connection.

The present invention also provides a compact body-wearable, anatomically-shaped, monitoring device intended for use by a ambulatory user which enables the measurement, processing, analysis and onward transmission of multiple physiological parameters where said device comprises:
a sensor electronics module unit which processes analyses and transfers the physiological information to remote device for capture, display, analysis or further processing either by wired or wireless means;
a body worn connection unit containing three or more electrodes arranged in an approximately triangular configuration of which the upper point is placed approximately at the sternum and the lower points approximately on the abdomen;
   and is able to measure and process;
two or more distinct views of the user's electrocardiogram (ECG);
respiration effort by measuring electrical impedance or motion changes;
skin surface temperature;
body gravitational load in vertical and horizontal axes; and/or
skin surface electrical impedance.

The sensor electronics module is anatomically-shaped to fit the users thoracic cavity in an approximately triangular, three-lobed arrangement, and shaped to fit between the sternum and abdomen.

The sensor connection unit consists of a central connection conformal material piece shaped to fit between the users sternum and abdomen in an approximate triangular, three-lobed configuration. The central connection piece contains the means to connect and support the sensor electronics module by three or more electrically conductive snap rivet fittings and, in turn, connects these fittings to three or more body contacting conductive electrodes and sensors. It is retained in place by a horizontal flexible fabric strap and two vertical fabric straps extending from the sternum point over each shoulder and reconnecting to the horizontal strap where the two meet on the user back.

The sensor electronics module contains an additional high density electrical interconnect terminal which may be connected to, via a male electrically conductive set of spring contacts of similar dimensions, held within the body worn connection unit and enables the connection of:
wired computing terminals.

Based on an appropriate algorithm within the sensor, an overall indication of predicted welfare of a person is produced, which removes the need for the device, by default, to send the physiological signals from the user's body to a remote unit for analysis and review. If the device determines that the physiological signals are abnormal in some way, perhaps indicating that the subject is over-exerting himself or herself, or that they have been injured or incapacitated in some way, then the device signals this to the monitoring point and can either automatically or on request from the monitoring point transmit additional physiological data and signals itself, for further analysis. For a group of users, it is possible to route data that falls into this category appropriately to a healthcare practitioner, reducing the number of such healthcare practitioners needed and optimising how this valuable expertise is best used.

The ability of the invention to determine whether or not to transmit the physiological data has three advantages. Firstly, it reduces the bandwidth, under normal conditions, that is needed to transmit the information. Secondly, because, under normal conditions, it does not have to transmit very much information, the transmitter needs to be turned on only for only a short time, at either frequent or infrequent intervals. This reduces the power consumption of the device and, accordingly, increases the battery life. Thirdly, it aids the rapid identification of users who may need more detailed observation, particularly when prioritising care amongst a group of users is necessary.

Advantageously, the invention provides a battery powered body-wearable part capable of collecting a plurality of physiological signals whilst minimising the size and area of the body covered by the device. In particular, the device does not rely on the need to site distributed sensor devices on the user's body in order to gain access to the signals needed. Hence, the chances of interfering with other clothing or equipment worn by the user is significantly reduced.

The device consists of a sensors module which is directly attached and supported by a single sensor connection assembly arranged in an approximately triangular shape to fit on and around the thoracic cavity and contains a plurality of sensors which in conjunction with the sensor module provides one of more of the following signal measurements:
- two or more distinct views of the users electrocardiogram (ECG), those skilled in the art will be aware that the provision of two or more distinct electrical views of the heart allows an improvement in the detection and accuracy of heart beat electrical activity, by allowing the electrical activity to be compared on each view, and improved immunity to noise which is more prevalent;
- respiration effort derived from electrical impedance changes within the body, because of thoracic cavity and abdominal movement, which occurs during breathing.
- Respiration Effort derived from directly measuring thoracic cavity expansion and contraction;
- sp0₂ blood Oxygen and pulsatile waveform extraction by measuring the blood oxygenation variations above the user's sternum;
- thoracic cavity skin surface impedance
- Correct electrode body contact confirmation by measuring impedance between electrodes.
- Skin surface temperature; and/or
- activity, impact and body position levels derived from 2 or 3 orthogonal axes of gravitational force measurement made using accelerometer devices contained in the sensor module.

The sensor electronics module is self-supported by the sensor connection device and can be easily mounted and dismounted from the sensor connection device without the need for special tools, by the use of suitable connectors, such as, conductive press-rivets. This aids washing and general maintenance of the sensor.

The shape and ergonomics of the sensor connection device are such that the user can correctly apply the device without the need for specialist medical assistance and can be used by both male and female users.

The monitoring device collects and analyses those signals, computes signal rates and periodicities, and provides an indication of the user's welfare status over a communications link, which may be radio or wire, and which may be constrained to be low bandwidth. This transmission may also dynamically contain the signals and analyses used to derive this welfare status.

The assessment of a person's welfare status needs to be robust in order to minimise the risks of false or missed alerts. Ambulatory activities can produce significant noise and environmental influences which may degrade the signals measured and the monitoring device has extended tolerance to such factors by the selection of methods and signal processing. In addition, defective or degraded operation of the device needs to be identified and conveyed back to the monitoring station in order to allow the monitoring station to indicate a measure of confidence in the data being displayed. Thus, the device contains more that one method of measuring physiological signals of high importance, for example, heart and respiration effort rate and can cross-check the measured rates (from the different methods) as well as the individual signal quality, in terms of possible noise content, and uses this to derive an overall confidence score. This confidence score may be used to inform the welfare indication score and to indicate to the remote user when the indication score may be considered unreliable. Such additional measures may adversely affect the power consumption of the device and to minimise this, the device shall have the means of dynamically switching on and off electronics and software processing of such secondary measures internally, such that they are only powered on when the recorded physiology becomes close to an abnormal classification or periodically as a safety measure. In addition, optimal sensitivity and fidelity of the measurement of cardio respiratory signals will vary depending on whether the user is moving or not moving. For example, during rest, the user's breathing effort will reduce in frequency and level and require more sensitive analysis. During activity, especially high activity, these settings would be sub-optimal and make the device prone to the effects of noise. The device copes with this by altering the sensitivities and signal bandwidths used on the physiological signals based on the sensors own measurement of the users, ambulation and activity status, as well as body position.

The device provides an indication of a user's welfare by a simple enumerated score. The basic welfare of the user is assessed by the measurement of primary cardio respiratory vital signs. The users heart and respiration rate are continuously computed by the sensor and compared against a variety of thresholds and time periods specific to potential indications of initial or advanced trauma. This may be optionally supplemented by the measurement of a user's blood oxygen content using the known technique of pulse oximetery, which can provide additional information on the user's cardio respiratory function.

Differentiation in the cardio respiratory score is given between normal and elevated vital signs, for example, an excessively high or low heart rate, and the absence of a rate which may indicate an urgent life threatening situation. Furthermore, the absence of cardio-respiratory vitals signs for an extended period of time is also scored explicitly in order to assist a remote system operator in gauging the priority of who to attend to first. As such rates may, in normal life, vary significantly, depending on at least the user's activities, the sensor uses a measure of the users general activity, calculated within the sensor, to modify thresholds used to determine normal or abnormal signs to account for this. Importantly, the score needs to also include a means to indicate unknown status owing to, for example, low confidence in the underlying vital signs signals captured or to an internally detected sensor malfunction.

Those skilled in the art of trauma medicine will be aware that the healthcare practitioner achieves an informed assessment of a user's welfare and prognosis by using a mixture of measurements of the users physiology and the users physical and mental status when physically examined. Such assessment assists the practitioner in allocating priority and deciding on the optimal timing for any intervention. Lack of physical proximity to a suspected casualty prohibits this and, henceforth, an important feature of the device is its ability to sense certain additional information which may be used to modify the users current cardio, respiratory score and to better inform the personnel at the monitoring station by providing such additional contextual information.

Contextual information may include, for example, the users body position, the users current and recent activity levels, evidence of the users ambulation, evidence of transient high gravitational shock load to a users body, and also the users current cognitive status. The latter is measured by the sensor requesting the user to perform an action and determining whether this has been undertaken correctly. Those skilled in the art will be aware that a positive result on the later presents a significant measure of trauma severity and prognosis. Furthermore, additional granularity can be achieved by the user differentiating such a response to indicate their personal status, for example, assistance needed/not needed. This feature provides an additional safety mechanism for this system and user as well as dealing with any unforeseen equipment malfunction which may affect the body electrical and mechanical signals used to derive the cardio respiratory data.

Those skilled in the art, will also be aware that other forms of injury may occur which do not necessarily result in immediately abnormal cardio, respiratory data, for example, loss of consciousness or the onset of thermal injury due to work intensity and environment. The device provides a secondary assessment of a user's neurological and thermal welfare status. The user's neurological status, measured by the method discussed above, may be ascertained for example, periodically, on detection of excessive body g-shock, on certain body positions and activity levels, combinations of these events and on demand from the monitoring station. The measurement of thermal status often relies on measuring a user's core body temperature or an estimate of the user's core body temperature and comparing this result against established guidelines, to determine the user's thermal status. Such methods are often invasive and socially unacceptable outside a clinical environment. As an example, a simple thermistor may be used.

Those skilled in the art will be aware that several defined indices exist to estimate and score the physiological strain a user is under and to equate this to a prediction of a user's heat strain. These indices rely, as a minimum, on the measurement of skin temperature made with an insulated skin temperature probe, to reduce the effects of environmental factors on the measurement and also on the users heart rate, as this will be seen to increase with increased core body temperature and can also provide an indication of the users work intensity. Hence the sensor incorporates the means to measure and compute such an index.

The sensitivity and specificity of the design is important in determining its latency in determining physiological changes considered potentially abnormal and those skilled in the art will be aware that this may be improved if the thresholds and rates which the welfare score uses can be specific to the user and not based on occupational analysis. Hence a further important feature of the device is for these thresholds to be trained to a user. Such a training modality can be triggered by a command to the device. The user can then be asked to perform a series of activities from which the sensor records and updates such threshold information held within it in a non-volatile memory. Such a training mode may be used as part of, for example, an annual fitness assessment or occupational training refresher course for a user. The information may also be provided to the device by independent measurement and then transferred into the device by an appropriate transfer means, for example, from a computing terminal or a personally-worn electronic record or tag.

Advantageously, an important feature of the system is that, either on remote request, or automatically the device can provide the raw physiological signals to the monitoring station. On identification of a possible casualty, this data may then be passed to a health care practitioner, either locally or remotely to the user to assist in determination of an appropriate course of action. In order to achieve a reasonable battery life which is an important feature for a remotely worn device, and also to minimise the system transmission load for the sensor, it uses a multilevel control on the way data is transferred. Accordingly, and advantageously, the monitoring device is capable of operating under 'abbreviated disclosure' or 'full disclosure'.

'Abbreviated disclosure' is intended to be the normal mode of operation for the sensor when attached to a healthy user. In abbreviated disclosure, system data need only be transferred in short bursts (say every thirty seconds or so). However, it may be desirable to transfer data every couple of seconds or at, say, fifteen second intervals. The data that could be transferred in this message is likely to contain:
- a primary and secondary welfare indication score for a user;
- a user's heart and respiration rate;
- the skin temperature of a user;
- the motion / activity level of a user;
- the body orientation of a user;
- unit / user identification information; and/or
- unit self-check diagnostics (lead off signal, battery status, etc.).

This data would be in the order of a few bytes and presents a very low transmission load on the system. The use of abbreviated disclosure therefore results in a substantial reduction of both transmission bandwidth and of power consumption.

'Full disclosure' is entered into on detection of a change in the welfare index score which may indicate a need for medical attention, or be triggered manually via a protected button, which could be operated by a distressed user or by a medic, or by remote request from the monitoring device. Once switched to 'full disclosure' the system could remain in that mode, or go back to 'abbreviated disclosure' after a time period or the cessation of the triggering event. The signals provided in 'full disclosure' may vary depending on system preferences and may also be controlled by the remote monitoring station.

Preferably, the body worn device is able to communicate, by wired or wireless means, with a radio communication device ,such as a GSM mobile phone; a satellite communication device; or public safety communication device (e.g. TETRA radio), in order to allow data transfer from a user over a wide area back to the monitoring station. The communication link may also be used to issue commands and configuration data to the device from the remote monitoring station. As such, the communication is preferably two-way.

Additionally, the present invention provides the ability for the user to be monitored from close proximity by communication with a remote hand-held communications device, for example, a Pocket PC, with an appropriate function to view the data.

In an alternative embodiment, for a user who may have sustained an injury, an attending healthcare practitioner may require unimpeded access to a user's body and also may benefit from the ability to select specific signal pick up points on the body in line with established medical practises or the type of injury being assessed. Hence, the same sensor device may be removed from the body and reconnected using appropriate cables to standard medical electrodes and can continue to offer the health care practitioner physiological data information during treatment.

The term 'ambulatory', as used herein, means capable of moving or actually moving, for example, of or pertaining to walking.

The following clauses describe further preferred aspects of the present invention:
1.) A monitoring device wearable by a person to be monitored, comprising:
   one or more sensing means for sensing cardio, respiratory, physiological and/or other information from the person;
   processing means for analysing the sensed information;
   memory means for storing the sensed and/or analysed information; and
   communication means for transmitting at least a portion of the analysed information, wherein:
      at least one waveform acquired from the sensed cardio, respiratory, physiological and/or other information is digitised in real-time;
      analysis of the sensed and/or digitised information is performed in real-time and a welfare indication of the person computed in real-time; and
      the computed welfare indication of the person is transmitted by the communication means and/or stored in the memory means.
2.) A monitoring device as set forth in clause 1, wherein the communication means is capable of transmitting sensed information.
3.) A monitoring device as set forth in clause 1 or clause 2, wherein the communication means is capable of transmitting digitised information.
4.) A monitoring device as set forth in any preceding clause, capable of transmitting the welfare indication, the sensed information and/or the digitised information in real-time.
5.) A monitoring device as set forth in any preceding clause, capable of transmitting the welfare indication every 0.0001 to 60 seconds, more preferably every 5 to 30 seconds, and ideally every 15 seconds.
6.) A monitoring device as set forth in any preceding clause, wherein the communication means is part of a radio, satellite and/or other communications network.
7.) A monitoring device as set forth in any preceding clause, wherein all waveforms acquired from the sensed cardio, respiratory, physiological and/or other information are digitised in real-time.
8.) A monitoring device as set forth in any preceding clause, wherein all sensed information is digitised in real-time.
9.) A monitoring device as set forth in any preceding clause, wherein the sensing means comprises one or more skin electrodes.
10.) A monitoring device as set forth in clause 9, wherein the sensing means comprises one or more electrodes and associated electronics circuitry.
11.) A monitoring device as set forth in any preceding clause, wherein the one or more sensing means comprises at least two sensing means.
12.) A monitoring device as set forth in any preceding clause, wherein at least part of the memory is a buffer-type memory.
13.) A monitoring device as set forth in any preceding clause, wherein the processor is capable of processing at least two forms of information selected from cardio, respiratory, physiological and/or other information, to derive data relating to a welfare indication of a wearer.
14.) A monitoring device as set forth in any preceding clause, wherein the processor is capable of processing the (primary) cardio, respiratory, physiological and/or other information to derive secondary cardio, respiratory, physiological and/or other information and the processor is capable of processing at least two forms of information selected from the primary and/or secondary cardio, respiratory, physiological and/or other information to derive data relating to a welfare indication.
15.) A monitoring device as set forth in any preceding clause, wherein the monitoring device comprises a plurality of integrated sensors for detecting the cardio, respiratory, physiological and/or other information.
16.) A monitoring device as set forth in any preceding clause, wherein the welfare indication is determinable by analysis and/or comparison of newly sensed cardio, respiratory, physiological and/or other information with thresholds from configurable data stored in the memory.
17.) A monitoring device as set forth in any preceding clause, wherein the monitoring device is capable of detecting cardio, respiratory, physiological and/or other information relating to one or more of the following:
   a) an electrical view of the heart of a person;
   b) the respiration effort of a person;
   c) the blood oxygen level of a person;
   d) the skin surface impedance of a person;
   e) whether there is correct skin electrode and person contact;
   f) the skin surface temperature of a person;
   g) whether a specific activity is being undertaken by a person;
   h) whether a person has been effected by an impact;
   i) the body orientation of a person;
   j) the movement of a person;
   k) the level of ambulation of a person;
   l) the absence of expected data;
   m) the cognitive state of a person;
   n) a person's own assessment of welfare; and/or
   o) whether excessive gravitational forces are being exerted on a person.
18.) A monitoring device as set forth in clause 16 or clause 17, wherein the thresholds and configurable data are automatically, manually or remotely modifiable or learned for a specific person.
19.) A monitoring device as set forth in any one of clauses 16 to 18, wherein the thresholds and configurable data are modifiable for a type of range of activities or environments.
20.) A monitoring device as set forth in any one of clauses 16 to 19, wherein the thresholds and configurable data are modifiable as a result of contextual information relating to a person.
21.) A monitoring device as set forth in any one of clauses 16 to 20, wherein the configurable data is derivable from previous analysis and/or comparison of cardio, respiratory, physiological and/or other information and the thresholds.
22.) A monitoring device as set forth in any one of clauses 16 to 21, capable of providing the configurable data from analysis of time-thresholds which conditions must be measured before a transition in the welfare indication occurs for one or more of the following conditions:
   a) high, low or intermediate signal rates;
   b) an absence of measurable signal rates;
   c) the rate of change of an averaged signal rate;
   d) averages of a measured signal rate;
   e) the short-term average of a measured signal rate;
   f) the long-term average of a measured data signal rate;
   e) the normal or abnormal characteristics of a waveform; or
   f) intermediate average of a measured signal rate;
   g) the time-threshold periods for transitions and/or average windows.
23.) A monitoring device as set forth in any preceding clause, wherein the welfare indication is capable of being overridden or reduced in severity by additional contextual information experienced by a person.
24.) A monitoring device as set forth in clause 23, wherein the contextual information relates to one or more of the following:
   a) whether a person is moving;
   b) whether a person has been effected by an impact;
   c) whether a person is carrying out a specific activity;
   d) the current or recent level of ambulation of a person;
   e) environmental factors experienced a person; or
   f) the cognitive state of a person.
25.) A monitoring device as set forth in clause 24, wherein the environmental factors include:
   a) ambient temperature;
   b) ambient pressure;
   c) altitude;
   d) humidity; or
   e) relative motion of the person.
26.) A monitoring device as set forth in any preceding clause, wherein the sensitivity of detection is modifiable in response to the activity status, level of ambulation and/or body position detected by the monitoring device, and/or contextual information experienced by a person.
27.) A monitoring device as set forth in any preceding clause, wherein the monitoring device is capable of sensing more than one measurement of cardio information.
28.) A monitoring device as set forth in clause 27, capable of detecting information relating to two distinct measurements of heart rate.
29.) A monitoring device as set forth in clause 27 or clause 28, wherein the measurements are provided by analysis of a person's ECG waveform, and/or a second alternative view of a person's ECG waveform and/or pulse train, using R-wave analysis and/or analysis of a person's blood oxygen pulsatile waveform.
30.) A monitoring device as set forth in any preceding clause, capable of detecting more than one measurement of respiratory information.
31.) A monitoring device as set forth in clause 30, capable of detecting information relating to three distinct measurements of respiration rate.
32.) A monitoring device as set forth in clause 31, wherein measurements are provided by chest expansion measurements, thoracic impedance pleythismography measurements and from measurements of electrocardiograph data.
33.) A monitoring device as set forth in any one of clauses 27 to 32, capable of comparing the more than one measurement of cardio information to provide a cardio confidence score.
34.) A monitoring device as set forth in any of clauses 30 to 33, capable of comparing the more than measurement of respiratory information to provide a respiratory confidence score.
35.) A monitoring device as set forth in clause 33 and clause 34, capable of analysing the cardio confidence score and the respiratory confidence score, together with data relating to the individual signal quality or contextual information to provide an overall confidence score.
36.) A monitoring device as set forth in any preceding clause, wherein the welfare indication is selectable from:
   normal; low priority alert; high priority alert; and unknown/un-operative.
37.) A monitoring device as set forth in clause 36, wherein the welfare indication comprises an additional state of:
   absence or substantial absence of vital signs.
38.) A monitoring device as set forth in any preceding clause, wherein the monitoring device is capable of modifying the severity of its welfare indication and the time threshold for indicating the welfare indication following detection of the absence, or substantial absence, of one or more cardio or respiratory measures.
39.) A monitoring device as set forth in any preceding clause, wherein, when a person has initially a normal welfare indication, a second cardio and/or respiratory measurement is triggerable automatically following determination of an abnormal welfare indication.
40.) A monitoring device as set forth in any preceding clause, wherein, when a person has initially a low-level abnormal welfare indication, a second cardio and/or respiratory measurement is triggerable automatically following determination of a progressively abnormal welfare indication.
41.) A monitoring device as set forth in any preceding clause, wherein the welfare indication provides the monitoring station with grades of normality (normal) and abnormality (inactive/in-operable or absence/substantial absence of vital signs) of the health (welfare) of a person.
42.) A monitoring device as set forth in any preceding clause, wherein a secondary welfare indication is provided by analysis of thermal and/or neurological information.
43.) A monitoring device as set forth in clause 42, wherein the cognitive state of a person is manually determinable by the monitoring station requesting the wearer to carry out an action.
44.) A monitoring device as set forth in clause 42 or clause 43, wherein the cognitive state of a wearer is automatically determinable following: a variable or set time period; an abnormal welfare indication; or evidence of excessive g-shock to a person, by the person being automatically requested to carry out an action.
45.) A monitoring device as set forth in clause 43 or clause 44, wherein the person is requested by visual, audible, vibrational or other sensory means.
46.) A monitoring device as set forth in clause 45, wherein the frequency of request can be varied depending upon the detection of a response or the type of response of a person.
47.) A monitoring device as set forth in any one of clauses 43 to 46, wherein an abnormal welfare indication is cancellable or movable towards normal by a person responding to the request to carry out the action.
48.) A monitoring device as set forth in any one of clauses 43 to 46, wherein the action of the person is capable of modifying the welfare indication to indicate a worsening of his/her welfare.
49.) A monitoring device as set forth in any one of clauses 43 to 46 or clause 48, wherein the action of the person indicates that assistance is required.
50.) A monitoring device as set forth in any preceding clause, capable of providing a secondary welfare indication by analysis of a measure of physiological strain derivable from a function of heart rate of the person and the insulated skin temperature, and configurable data stored in the memory.
51.) A monitoring device as set forth in any preceding clause, wherein the monitoring device is capable of abbreviated disclosure, when only a subset of the digitised information is communicated to the monitoring station, or full-disclosure, when all digitised information is communicated to the monitoring station.
52.) A monitoring device as set forth in clause 51, wherein, under full-disclosure, some or all of the waveforms of the cardio, respiratory, physiological and/or other information may be transmitted to the monitoring station.
53.) A monitoring device as set forth in clause 51 or clause 52, wherein full-disclosure can be activated automatically by determination of an abnormal welfare indication.
54.) A monitoring device as set forth in clause 51 or clause 52, wherein full-disclosure is manually-activatable by a person or by the monitoring station.
55.) A monitoring device as set forth in any one of clauses 51 to 54, wherein the subset comprises one or more of:
   a) primary and/or secondary welfare indication;
   b) heart and/or respiration rate;
   c) skin temperature;
   d) motion and/or activity level;
   e) body orientation;
   f) user identification information;
   g) unit identification information;
   h) unit self-check diagnostics; and/or
   i) confidence scores.
56.) A monitoring device as set forth in any preceding clause, further comprising a request and response device, wearable by a person, for communication with the monitoring device and/or the monitoring station.
57.) A monitoring device as set forth in clause 56, wherein the request and response device is wrist-worn.
58.) A monitoring device as set forth in clause 56 or clause 57, wherein the request and response device comprises one or more sensors and/or a watch.
59.) A monitoring device as set forth in clause 58, wherein the sensors comprise a heart rate sensor and/or an accelerometer.
60.) A monitoring device as set forth in any preceding clause, capable of transmitting the welfare indication, the digitised cardio, respiratory, physiological and/or other information or the waveforms of the cardio, respiratory, physiological and/or other information direct to a monitoring station, or via intermediate transfer or monitoring equipment.
61.) A monitoring device as set forth in any preceding clause, wherein assessment of a person's welfare is optimised by transmittal and storage of wearer-personalisation information, environment information and/or activity information by the monitoring station, any intermediate equipment and/or the monitoring device.
62.) A monitoring device as set forth in any preceding clause, comprising two-way communication means between it and a monitoring station or intermediate equipment.
63.) A monitoring device as set forth in clause 62, wherein the monitoring device comprises a wireless transmitter and receiver for communication with the monitoring station or intermediate equipment.
64.) A monitoring device as clamed in clause 62, wherein communication between the monitoring device and the monitoring station or intermediate equipment is provided by a wired connection.
65.) A monitoring device as set forth in any preceding clause, further comprising connectable external sensors for detection of further cardio, respiratory, physiological and/or other information.
66.) A monitoring device as set forth in clause 65, wherein the external sensors communicate by wired or wire-less connections.
67.) A monitoring device as set forth in any preceding clause, capable of detecting the presence of motion of a person and using the evidence of motion to reduce the bandwidth of the cardio signal receiver to improve the signal to noise ratio and improve performance.
68.) A monitoring device as set forth in any preceding clause, capable of detecting the presence of motion and body position of a person and using evidence of motion and body position to modify the signal gain, bandwidth and sensitivity of the respiratory signal receiver to improve performance.
69.) A monitoring device as set forth in any one of clauses 62 to 68, wherein the monitoring station is capable of uploading to the monitoring device contextual information and/or configurable information.
70.) A monitoring device wearable by a person to be monitored, comprising:
   a detachable anatomically-shaped sensor electronics module comprising processing means, memory means and communications means; and
   a connector, harness and/or other support wearable by a person, capable of attaching, or holding in sensory/sensing proximity, the sensor electronics module to a person, and comprising one or more sensing means, wherein the monitoring device:
   senses cardio, respiratory, physiological and/or other information from a person; and
   performs real-time analysis of the sensed information and computes a real-time welfare indication of the person for onwards transmission / communication.
71.) A monitoring device as set forth in clause 70, wherein the one or more sensing means are arranged to provide electrical/electronic communication with an attached sensor electronics module.
72.) A monitoring device as set forth in clause 70 or clause 71, wherein the one or more sensing means comprise at least two sensing means.
73.) A monitoring device as set forth in any one of clauses 70 to 72, wherein the sensing means comprises one or more skin electrodes.
74.) A monitoring device as set forth in clause 73, wherein the sensing means comprises one or more skin electrodes and associated electronics circuitry.
75.) A monitoring device as set forth in any one of clauses 70 to 74, wherein the communication means is part of a radio, satellite and/or other communications network.
76.) A monitoring device as set forth in any one of clauses 70 to 75, wherein the monitoring device comprises means for detecting skin temperature.
77.) A monitoring device as set forth in clause 76, wherein the means for detecting skin temperature is a thermistor.
78.) A monitoring device as set forth in any one of clauses 70 to 77, wherein the monitoring device comprises means for detection of motion, body position and/or impact.
79.) A monitoring device as set forth clause 78, wherein the means for detection is an accelerometer.
80.) A monitoring device as set forth in any one of clauses 70 to 79, wherein the monitoring device further comprises a chest-expansion sensor.
81.) A monitoring device as set forth in clause 80, wherein the chest-expansion sensor is a variable strain sensor.
82.) A monitoring device as set forth in clause 80 or clause 81, wherein the chest expansion sensor is provided as part of a yolk.
83.) A monitoring device as set forth in any one of clauses 70 to 82, wherein the monitoring device comprises means for detecting blood oxygen levels of a user.
84.) A monitoring device as set forth in clause 83, wherein the means for detecting blood oxygen levels is a reflectance-type sensor for pulse oximetry analysis.
85.) A monitoring device as set forth in any one of clauses 70 to 84, wherein the sensor electronics module is capable of real-time analysis of information to provide a welfare indication of a person.
86.) A monitoring device as set forth in any one of clauses 70 to 85, wherein the sensor electronics module is capable of acquiring, storing and digitising the waveform of the cardio, respiratory, physiological and/or other information for internal analysis and/or onwards transmission.
87.) A monitoring device as set forth in any one of clauses 70 to 86, wherein the sensor electronics module is capable of onwards transmission to a monitoring station.
88.) A monitoring device as set forth in any one of clauses 70 to 87, wherein the sensor electronics module is capable of onwards transmission to intermediate equipment.
89.) A monitoring device as set forth in clause 88, wherein the intermediate equipment is a further transmission device or a portable computer.
90.) A monitoring device as set forth in any one of clauses 86 to 89, wherein the sensor electronics module is capable of communication by wired or wireless means.
91.) A monitoring device as set forth in any one of clauses 70 to 90, wherein the sensor electronics module is capable of measuring, processing, analysing and/or onwards transmission of information relating to one or more of the following:
   a) an electrical view of the heart of a person;
   b) the respiration effort of a person;
   c) the blood oxygen level of a person;
   d) the skin surface impedance of a person;
   e) whether there is correct skin electrode and person contact;
   f) the skin surface temperature of a person;
   g) whether a specific activity is being undertaken by a person;
   h) whether a person has been effected by an impact;
   i) the body orientation of a person;
   j) the movement of a person;
   k) the level of ambulation of a person;
   l) the absence of expected data;
   m) the cognitive state of a person;
   n) a person's own assessment of welfare; and/or
   o) whether excessive gravitational forces are being exerted on a person.
92.) A monitoring device as set forth in any one of clauses 70 to 91, wherein the sensor electronics module is capable of measuring, processing, analysing and/or onwards transmission of more than one measurement of cardio information.
93.) A monitoring device as set forth in clause 92, wherein the sensor electronics module is capable of measuring, processing, analysing and/or onwards transmission of two distinct views of a person's electrocardiogram.
94.) A monitoring device as set forth in any one of clauses 70 to 93, wherein the sensor electronics module is capable of measuring, processing, analysing and/or onwards transmission of more than one measurement of respiratory information.
95.) A monitoring device as set forth in clause 94, wherein the sensor electronics module is capable of measuring, processing, analysing and/or onwards transmission of chest expansion measurements, skin impedance measurements and measurements from electrocardiograph data.
96.) A monitoring device as set forth in any one of clauses 70 to 95, wherein the sensor electronics module is anatomically-shaped to fit the thoracic region of a person.
97.) A monitoring device as set forth in clause 96, wherein the sensor electronics module is shaped to fit in the region of the sternum and upper abdomen of a person.
98.) A monitoring device as set forth in any one of clauses 70 to 97, wherein the sensor electronics module comprises three lobes in a triangular configuration.
99.) A monitoring device as set forth in any one of clauses 70 to 98, wherein the wearable monitoring device comprises three skin electrodes.
100.) A monitoring device as set forth in any one of clauses 70 to 99, wherein the skin electrodes are spaced as far apart as possible, in the context of a person's body size.
101.) A monitoring device as set forth in any one of clause 70 to 100, wherein the skin electrodes are spaced from 5cm to 15cm apart.
102.) A monitoring device as set forth in clause 101, wherein the skin electrodes are spaced 10cm apart.
103.) A monitoring device as set forth in any one of clauses 70 to 102, wherein the connector harness and/or other support comprises one or more of the following:
   a) an adhesive pad;
   b) a yolk;
   c) an item of clothing; or
   d) standard electrocardiograph adhesive skin electrodes.
104.) A monitoring device as set forth in clause 103, wherein the adhesive pad is anatomically-shaped to fit the thoracic region of a person.
105.) A monitoring device as set forth in clause 104, wherein the adhesive pad is shaped to fit in the region of the sternum and upper abdomen of a person.
106.) A monitoring device as set forth in any one of clauses 103 to 105, wherein the adhesive pad comprises three lobes in a triangular configuration.
107.) A monitoring device as set forth in clause 103, wherein the yolk comprises an adjustable band capable of being located around the thorax of a person.
108.) A monitoring device as set forth in clause 107, wherein the yolk further comprises an over the shoulder strap for preventing movement of the yolk.
109.) A monitoring device as set forth in clause 103, wherein the item of clothing is a tight-fitting vest or T-shirt.
110.) A monitoring device as set forth in any one of clauses 70 to 109, wherein the sensor electronics module is connectable to the connector harness and/or other support by conductive snap-rivet fittings.
111.) A monitoring device as set forth in clause 110, comprising at least three snap-rivet fittings.
112.) A monitoring device as set forth in any one of clauses 70 to 111, wherein the sensor electronic module comprises an electrical interconnect which enables connection of one or more of the following:
   wired computing terminals;
   auxiliary sensors;
   an auxiliary pulse oximetry module;
   a power source.
113.) A monitoring device as set forth in clause 112, wherein the sensor electronics module comprises a data link for connection of auxiliary sensors, monitoring equipment, transmission equipment or any auxiliary electrical equipment.
114.) A monitoring device as set forth in clause 113, further comprising auxiliary, connectable sensor equipment.
115.) A monitoring device as set forth in clause 113, wherein the auxiliary equipment is a reflectance-type sensor for pulse oximetry analysis.
116.) A monitoring device as set forth in clause 113 or clause 114, wherein the auxiliary electrical equipment is a request and response device.
117.) A monitoring device as set forth in clause 116, wherein the request and response device can alert a wearer.
118.) A monitoring device as set forth in clause 116 or clause 117, wherein a person can communicate with the sensor electronics module or monitoring station using the request and response device.
119.) A monitoring device as set forth in any one of clauses 116 to 118, wherein the device is wrist-worn.
120.) A monitoring device as set forth in any one of clauses 70 to 119, wherein onwards transmission of information from the sensor electronics module is provided by wired or wireless means.
121.) A monitoring device as set forth in any one of clauses 70 to 120, wherein the sensor electronics module comprises a two-way transmitter for communication with a monitoring station or intermediate equipment.
122.) A monitoring device as set forth in any of clauses 70 to 121, wherein a person to be monitored is an ambulatory person.
123.) A monitoring system for monitoring of one or more persons comprising:

a monitoring device as set forth in any one of clauses 1 to 69 or as set forth in any one of clauses 70 to 122, wearable by the or each person being monitored; and
one or more monitoring stations, wherein:
   the or each monitoring device is in communication with the one or more monitoring stations; and
   the one or more monitoring stations receive and monitor the computed welfare indication from the or each monitoring device to assess the wellbeing of each person being monitored.
124.) A monitoring system as set forth in clause 123, wherein the system is capable of abbreviated disclosure, when only a subset of digitised information is communicated to the monitoring station, or full-disclosure, when all digitised information is communicated to the monitoring station.
125.) A monitoring system as set forth in clause 124, wherein, under full-disclosure, some or all of the waveforms of the cardio, respiratory, physiological and/or other information may be transmitted to the monitoring station.
126.) A monitoring system as set forth in clause 124 or clause 125, wherein full-disclosure is activatable automatically by determination of an abnormal welfare indication.
127.) A monitoring system as set forth in clause 124 or clause 125, wherein full-disclosure is manually activatable by a wearer or by the monitoring station.
128.) A monitoring system as set forth in any of clauses 123 to 127, wherein the welfare indication is selectable from: normal; low priority alert; high priority alert; and unknown/un-operative.
129.) A monitoring system as set forth in clause 128, wherein the welfare indication comprises an additional state of: absence of vital signs.
130.) A monitoring system as set forth in any of clauses 123 to 129, capable of transmitting the welfare indication, the digitised cardio, respiratory, physiological and/or other information or the waveforms of the cardio, respiratory, physiological and/or other information to a monitoring station via intermediate transfer or monitoring equipment.
131.) A monitoring system as set forth in any of clause 123 to 130, wherein the monitoring station and monitoring device communicate in a two-way manner by wired or wireless means.
132.) A monitoring system as set forth in any one of clauses 123 to 131, wherein configurable parameters may be determined, and adjusted, recorded and stored within the monitoring device whilst in a 'training mode', for use when the monitoring device is not in a training mode.
133.) A monitoring device substantially as herein described, with reference to or as shown in the accompanying drawings.
134.) A monitoring system substantially as herein described, with reference to or as shown in the accompanying drawings.

In order that the invention can be fully disclosed, embodiments of the invention are described, by way of example only, with reference to the accompanying drawings, in which:
Figure 1 is a diagram showing a monitoring a system according to the present invention;
Figure 2a is a front view of a person, showing approximate location of a sensing means according to the present invention;
Figure 2b is a back view of a person of Figure 2a;
Figure 3a is a front view of a person, showing location of a sensor electronics module and connector (yolk) according to the present invention;
Figure 3b is a back view of a person of Figure 3a;
Figure 4 is a view of the sensor electronics module and connector of Figures 3a and 3b, showing the manner of connection therebetween;
Figure 5 is a view of the connector of Figures 3a and 3b, showing the location of electrical and physical connectors on a strap-based harness;
Figure 6 is an adhesive connection assembly according to the present invention, showing the location of electrical and physical connectors on the adhesive pad;
Figure 7a and 7b are view of items of clothing incorporating a sensor electronics module according to the present invention.
Figure 8 is a view of a sensor electronics module according to the present invention, as used by a healthcare practitioner or paramedic;
Figure 9 is a block diagram showing the operation of a monitoring device according to the present invention;
Figure 10 is a block diagram showing a state transition diagram of the monitoring device according to the present invention for a welfare indication derived from cardio, respiratory, physiological and/or other information (primary welfare indication); and
Figure 11 is a block diagram showing a state transition diagram of the monitoring device according to the present invention for neurological response and thermal welfare indication (secondary welfare indication).

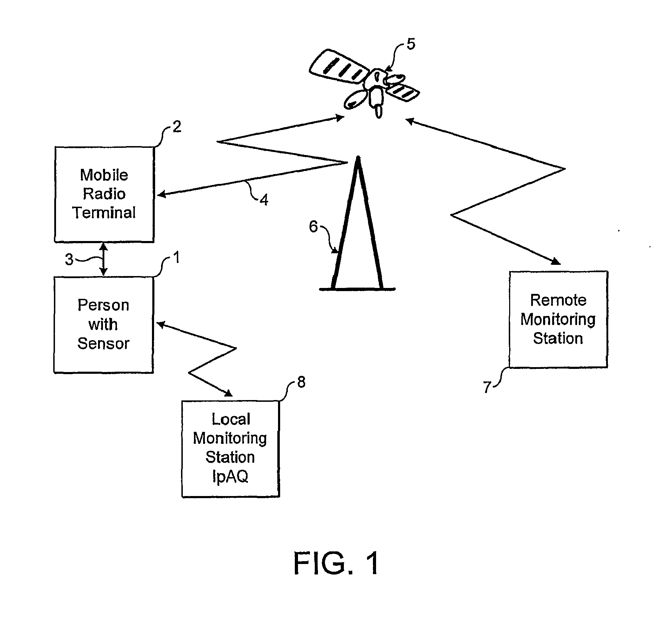

A monitoring system of the present invention is shown in Figure 1, in particular. A person or user wears a monitoring unit, the person and/or unit indicated generally by reference 1, which records multiple physiological signals from the user 1 and processes them in order to determine a welfare status (welfare indication). The user monitoring unit 1 communicates to a mobile radio terminal 2 via a communication link 3, in order to send data to, and receive data from the mobile radio terminal 2, which in turn communicates, via a communication link 4, to an infrastructure 4,5,6 to which a remote monitoring station 7 is connected. This provides remote access to a user to information from the user monitoring unit 1. The communication system may be, for example, a land-based mobile communications system, such as, a GSM mobile cellular network - which is shown by reference 6. Those skilled in the art will be aware that alternate networks, both terrestrial 6 and/or satellite 5, based may be used to transport the data to and from the remote user, at the monitoring station 7, and that the remote user may be, additionally, not in direct connection with the mobile communications network. In addition, a local monitoring station 8 may be used to communicate with the monitoring unit 1 directly, either by wired or wireless means. In a preferred embodiment, the local monitoring station 8 may be a hand-held computer 8, such as a Pocket PC.

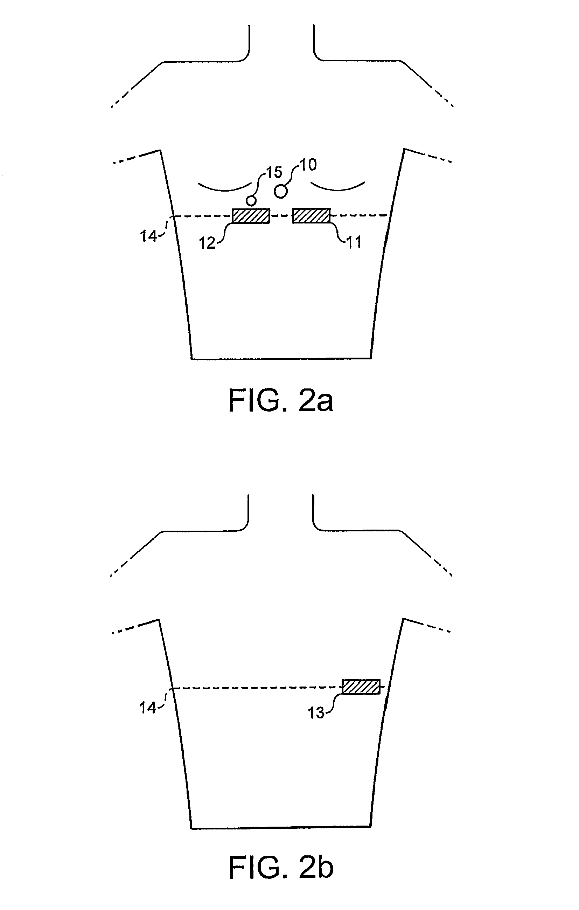

Figures 2a and 2b show respective front and back views of a user. The signals of interest may be derived from an approximately horizontal set of electrodes applied to the central thoracic cavity.

By the use of differential electrical amplification, the heart's electrical activity can be measured between electrode positions 11 and 12, 11 and 13, and 13 and 12. Those skilled in the art will be aware that, whilst the electrode spacing is small, for example 10 cm, the proximity of the sensor to the heart will compensate to allow a reasonable signal to noise ratio to be achieved. Respiration effort may be measured across electrodes 11 and 12, or 11 and 13 simultaneously, by presenting a high frequency AC signal from a constant current source, such that variation in the impedance of the diaphragm, owing to respiration, will result in a voltage waveform which approximates to respiration effort. This signal may be used to derive respiration rate after appropriate filtering.

The same technique can also be used to determine the impedance of the electrode 11, 12, 13 connection to the skin and to flag a "lead-off" condition if they exceed a certain threshold.

Those skilled in the art will also be aware that blood oxygen percentage level (SpO₂) and pulsatile waveform can also be measured using the established technique of pulse oximetry, and a reflectance-type sensor placed can be placed on the sternum bone within the same approximate area, as indicated by reference 10. The use of this sensor 10 may be optional depending on the user's requirements.

Skin surface temperature may be measured from a site close to reference 15, which is preferred because of its proximity to the user's liver.

Respiration effort may also be measured by the measurement of the rib cage expansion and contraction measured around some or all of the circumference of the thorax, as denoted by the dotted line referenced as 14. Those skilled in the art will know this measurement location to be consistent with a body function known as the 'zyphoid process', which can be used to derive respiration effort.

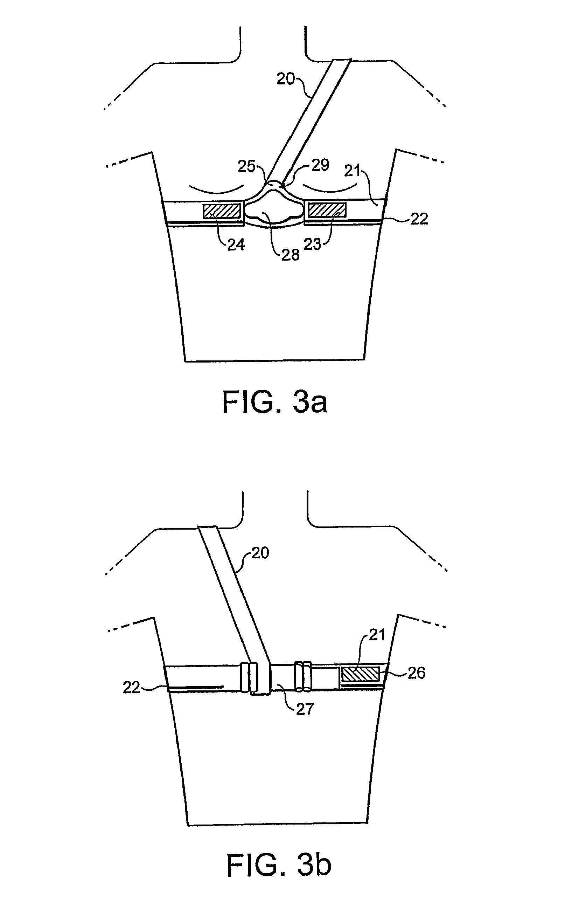

Figures 3a and 3b show the respective front and back of a user. The monitoring device electronics is housed in a unit 28 (sensor electronics module [SEM] 28) which is attached to a sensor connection harness 21. The sensor connection harness 20,21 contains within it, the necessary skin-contacting electrodes 23,24,25. These electrodes 23,24,25 can be made from silver coated fabric or a silver-loaded, silicon elastomeric block - as shown at reference 26. The harness 20,21 is held to the body firmly by an elastic waistband 21, which also contains a resistive (or variable) strain sensor 22 - which resistance changes with chest expansion. The horizontal band 21 is held in place by an over the shoulder strap 20, to reduce the chance of the harness 20,21 slipping down the torso during exercise. The tension on the horizontal strap 21 may be adjusted using an adjuster strap 27. The non-skin contacting side of the harness may be finished with a decorative fabric cover to protect the sensors within the strap. The harness 20,21 may be produced in varying sizes, for example small, medium and large, so as to cope with size variations of users. In addition, in the region of point indicated by reference 29, at which the strap 20 attaches to the central point 25, an aperture may be provided to place the reflectance pulse oximetry sensor in position above the sternum.

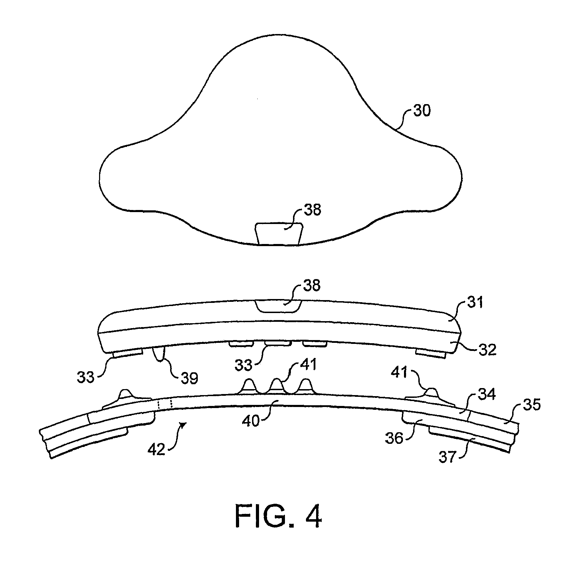

With reference to Figure 4, the SEM 30 is electrically and mechanically connected to a sensor connection assembly 42, which is secured to the body of a user using a strap-based harness 35. The sensor connection assembly 42 has a central mounting point (mounting plate) 40 made from, for example, a suitable non-conductive body-conformal material, for example, polycarbonate. The central mounting point 40 is attached, for example, by means of clothing stitching, to semi-flexible straps 35, which are passed around the body to secure the connection assembly 42 in place and hold the assembly 42 to the body with a degree on tension such that unwanted movement of the assembly is minimised. The SEM 30 is housed in a suitable plastic environmentally sealed enclosure 30 and can be designed to be compact, for example, around 73mm high by 123 mm wide by 16mm thick. The SEM 30 comprises an upper case 31 and lower case 32, which may be separated in order to fit the electronics hardware inside, as part of the manufacture of the SEM 30. The rear (or body-side) of the case also contains a skin probe 39 to contact the user's skin, in order to measure temperature. Electrical and mechanical connection is achieved using electrically conductive male snap-rivets 41 (for example Micron E391282-085 and E311-a2cl). The SEM 30 contains the matching female snap-fixings 33, allowing the module to be connected to the central mounting point by pressing the two parts together. An advantage of these snap-connections is that the unit may be separated with moderate hand pressure and, hence, can be done by the user when needed. Additionally, the SEM 30 provides an extra electrical interconnect interface 38 to allow charging of its internal battery, wired transfer of data, and connection to the pulse oximetry sensor. When not required, a moulded plastic bung (38) can be used to seal the connector interface.

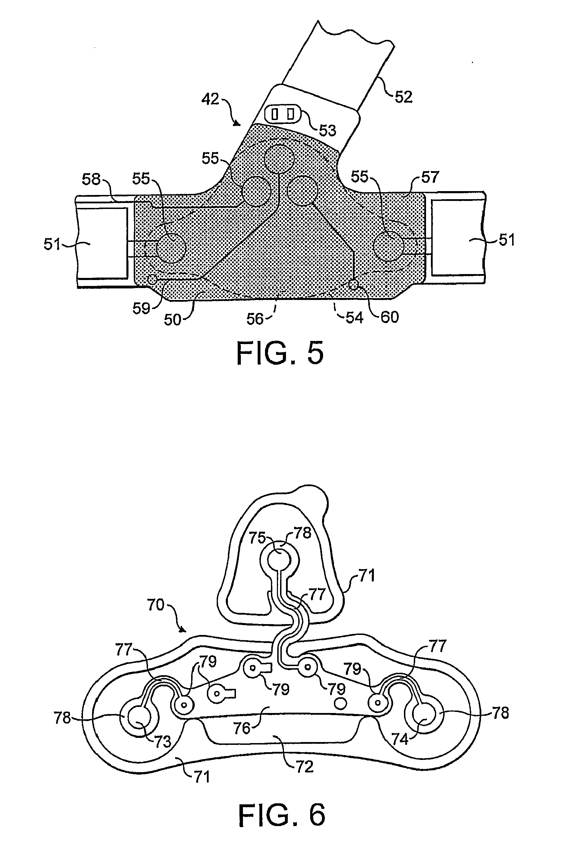

Figure 5 shows the body-facing side of the central mounting point 50,57 of the sensor connection assembly 42, showing part of an over the shoulder strap 52. The snap rivets 55 pass through the mounting point and allow electrical connection to the front electrodes 50,51. The third electrode and respiration band are connected by remote connection means 58. Those skilled in the art will be aware that this can be achieved by a number of means including, for example, flexible wires or flexible conductive printed circuit boards. The reflectance pulse oximetry sensor 53 is optionally held within the assembly 42 with an aperture to allow the sensor head to protrude and contact the body. It is electrically connected by wire to the SEM 30, the positioning of which is shown by the dotted line referenced as 54. Further electrical connectors are shown by references 59 and 60. Further, a protective, waterproof fabric layer 56 may be overlaid on the central mounting point 50 to cover the electrical connections and protect them from damage.

Figure 6 shows an adhesive sensor connection assembly 70. The use of an adhesive connection assembly is an alternative to the strap-based harness, discussed above. The assembly comprises a sculpted adhesive membrane 72 (for example the Intellicoat 5230 range) to hold the sensor to the skin of a user. The three electrodes 73,74 and 75 are provided by a circular hydrogel disk (for example Ludlow RG63B) of which one side contacts the skin and the other side is a flexible polyester membrane 76, printed with conductive silver /silver chloride ink tracks 77 for connecting between the electrode points 78 and the snap rivets 79, which are arranged in the same locations as used in the earlier strap harness example. 71 is a release liner material (e.g. Flexcon 94PRTPFW) used to protect the adhesive membrane before application to the body.

Those skilled in the art will also be aware that alternative electrode assemblies are commonly provided with conductive snap fittings, for example, Ambu® BlueSensor L, and that these could also be used with the present invention.

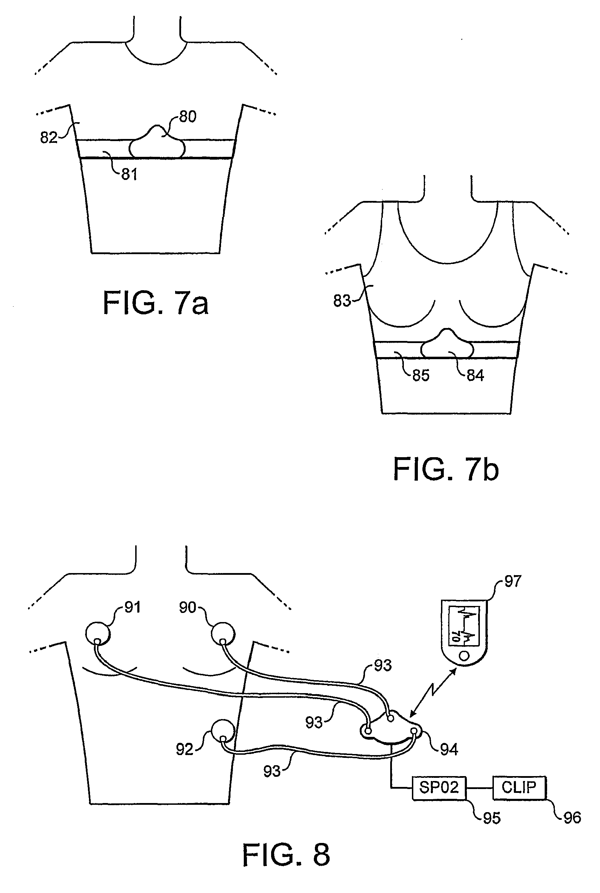

Figures 7a and 7b show further alternative ways of attaching the monitoring device to a user and show, in particular, how the monitoring device can be incorporated into a user's apparel, either as part of a male-user's vest 82 or as part of a female-user's vest 83. The vests 82,83 may be constructed from a suitable fabric, such a Lycra™, and have sewn internal to the vest electrodes of the style discussed earlier, such that it connects to the SEM 80, via the same conductive snap-rivet method discussed above. The vests 82,83 can also have integrated into them a flexible semi-conductive strap 81, as discussed earlier, to detect a user's respiratory chest movement.

Figure 8 shows diagrammatically how the monitoring device 94 can be remotely mounted from the user by a paramedic or healthcare practitioner. Commonly used ECG electrodes 91, 90 and 92 are used to connect to the users skin and, thus, to provide, through wires 93, an ECG signal view, as desired by the medic. For example, the configuration in Figure 8 provides an ECG view those skilled in the art will recognise as Lead I between 90 and 91 and Lead II between 91 and 92. Such conventional ECG views may offer an advantage of familiarity to a medic. Respiratory effort may also be measured between 91 and 92. The electrodes 90,91,92 connect to the SEM 94 by a special remote sensor connection device which has a connection plate comprising a plastic carrier and aforementioned conductive snap rivets connecting to flying electrode wires 93, with a suitable termination to connect to the electrodes 90,91,92. Additionally, the device contains a wired connection to a pulse oximeter device 95, for example, the NONIN XPOD, which provides a variety of sensor clip assemblies 96 to connect to the users body, for example, a finger, toe or ear clip. In this configuration, the medic may observe the sensor output by means of a portable computing device 97, for example an IPAQ, communicating to the sensor electronics module by wire or wireless means (for example Bluetooth™).

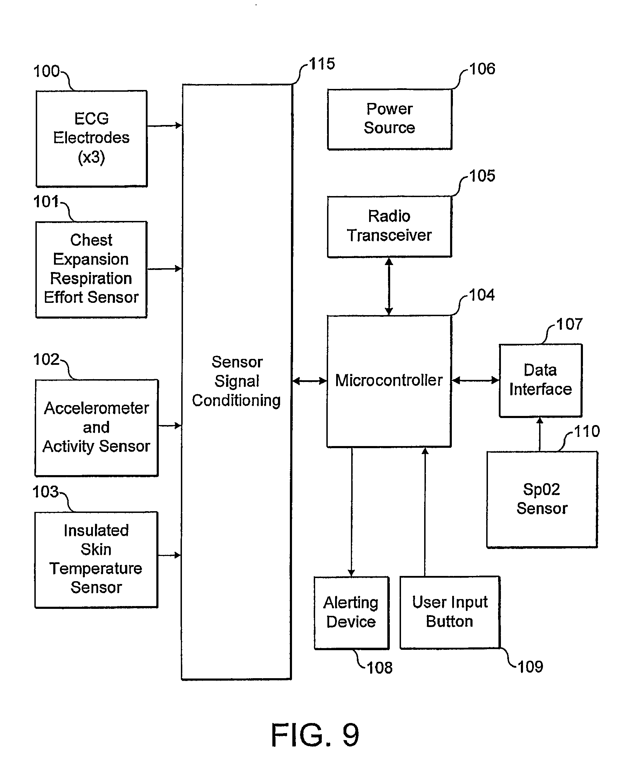

Referring to Figure 9, a preferred embodiment of the monitoring device of the present invention may be achieved as follows. ECG measurements are taken from the subject from electrodes sensors attached to the skin and connected to the electronics via connections 100. Considering a single channel of ECG, the ECG signal between two electrodes may be differentially amplified by an amplifier and filter stage contained in the signal conditioning circuit 115, greatly reducing the effect of noise, particularly mains electrical hum. After amplification, the ECG signal is
filtered using a band pass filter to select only those frequencies of interest. This is followed by further amplification and low-pass filtering before presentation of the signal to an analog to digital converter (A/D) input of the microprocessor unit 104, which may be an embedded microcontroller such as a Philips 80C51. Additional noise immunity may be provided by, for example, reducing the ECG bandwidth to, for example, 5Hz to 50Hz, when a user is moving and this may be controlled by the microcontroller, which is able to detect the presence of motion via the accelerometer 102. The additional ECG2 and ECG3 channels would be provided using the same methodology. One or more of the channels contained with the signal conditioning 115 can be have power switched to it by the microcontroller 104 in order to minimise power consumption when the additional signal is unnecessary. Once digitised the microcontroller 104 can performs additional filtering and thresholding specifically designed to detect the presence of the characteristics of an ECG waveform and from this data additional measures, such as ECG heart rate, by counting_the number of ECG pulses seen in a window. A signal quality measure can be provided by the microcontroller 104 by measuring the signal to noise ratio of the ECG waveform. Those skilled in the art will recognise several methods exist to undertake this computation within a microcontroller 104. Additionally, the same characteristics may be detected by a hardware circuit contained in 115, which is tuned to notch out the central energy contained in the ECG waveform. Those skilled in the art will recognise this as an R-wave detector. Such circuits have an advantage over the full-ECG derived method described earlier as they reduce power consumption, as the microcontroller receives only a single
logic pulse per heart beat and has to undertake less computation. The circuits have a disadvantage in that they are less sensitive to extreme low heart rates and do not adapt as well to a users specific ECG characteristics. Hence, R-wave analysis is also incorporated within the module to provide an alternate heart rate (HRr) and is used preferably when the user's physiology is well within normal expected values. A measure of signal quality can be derived from the R-wave pulse rate signal by measuring its periodicity which should be nominally regular. An overall confidence can then be derived, for example, a figure between 1 and 100, by the mathematical combination of the signal qualities and level of agreement of the two sensed heart rates. Those skilled in the art will recognise that several statistical and mathematical techniques exist to undertake such a computation. A chest expansion sensor is used to provide a primary method of measuring respiration effort (BRb). The sensor 101 is physically attached to the subject as part of the harness or assembly, and electrically connected as part of an impedance measurement network, with its centre point fed into an amplification stage 115 and a band pass filter. Further, amplification may be provided and low-pass filtering can be applied before presentation of the signal to an A/D input of the microprocessor unit 104. The level of gain may be dynamically switched by a logic line from the microcontroller 104 into the signal conditioning section and this may be set depending on the peak to valley levels measured by the microcontroller 104 or on other criteria such as body position. Once digitised by the microcontroller 104, it may deduce a rate by measuring peaks and troughs which will occur in relation to the breathing process. A signal quality indication can be derived from a combination of the symmetry of the breath peaks and troughs, the area of the breathing peak and the number of believed false breathing peaks detected. Respiration measurements may also be derived from the ECG signal. It is well-known in the art that, in a normal subject, the amplitude characteristic of the ECG signal varies over time, and this variation is associated with the respiration effort rate. The microcontroller contains an algorithm which measures this variation and then uses the derived signals to detect breathing peaks and troughs.

A third method to measure breathing rate is also employed which is impedance respiration effort, which is measured using a known technique called impedance thoracic pneumography. This is measured using a simple current source amplifier to drive an impedance signal to two of the ECG electrodes 100. The frequency of the current source amplifier output could be in the range of 50-150kHz. The impedance of the thoracic cavity will vary as the signal passes through it and the wearer breaths in and out. This variation will induce an amplitude change known as amplitude modulation to the constant current signal. The same electrodes (100) can be used to sense this voltage using a differential amplification stage contained in 115 and, after band-pass filtering a simple diode detector followed by further amplification and low-pass filtering, it can then be presented to an A/D input of the microprocessor unit 104. Breathing frequency detection can then be performed as discussed earlier. An overall confidence can also be derived by the microcontroller using similar techniques to those discussed for the heart rate. The preferred embodiment has provision for an accelerometer 102, which is assumed to be two orthogonally mounted two-axis devices, for
example the Analog Devices AD XL202E, but may also be a single three axis device. These devices provide the microcontroller 104, via an A to D port with a waveform indicating the g-force applied to the sensor. Thus, by suitable software processing, a body orientation may be deduced by the relative positions of each axes value and also activity and ambulation detected by the frequency and depth of the short term variation in each axes. In addition, high g-load may be measured by monitoring the short term peak value of the accelerometers output and, above a certain level, this signal can be used to assist the computation of welfare indication. Skin temperature is shown being measured by a simple thermistor 103, the output of which is amplified before being presented to an A/D input of the microprocessor unit 104. It will be clear to those skilled in the art that other methods of deriving the physiological parameters would be possible, and that other parameters could also be measured using well-known techniques. The monitoring device contains an alerting device 108 (request and response device 108) which can provide a vibrating sensation to the user in order to trigger a response from the user. Such devices are commonly used in mobile handsets to provide a covert alert and this may be advantageous in certain circumstances. Those skilled in the art will be aware that an audible or visual alert may also be easily incorporated into the sensor. A user's response can be measured by the operation of a button on the SEM or by asking the user to strike the SEM (monitoring device) and detecting the blow using accelerometers sensors contained within the SEM. The circuits described are powered by a cell or cells 106 which may be either primary (for example Alkaline LR03 cells) or secondary rechargeable (for example Varta LIP 553048), which may be regulated to provide a stable and controlled voltage to the circuit elements. After digitising information presented to it, the microcontroller 104 processes the signals further and may undertake further signal conditioning, filtering and numerical computation, in order to derive secondary measures from the signals, such as, rates. The device then uses this data to compute the welfare indication. The monitoring device sends the required data either to an rf transmitter 105, which may be, for example, a wireless transceiver such as a radio modem (for example, a Wireless Futures Bluewave™ or a Zigbee™ radio transceiver). Alternately, a wire-based communications driver 107 can be used. This communications driver 107 also provides a serial data communication interface for the connection of a pulse oximeter sensor 110 to the monitoring device.

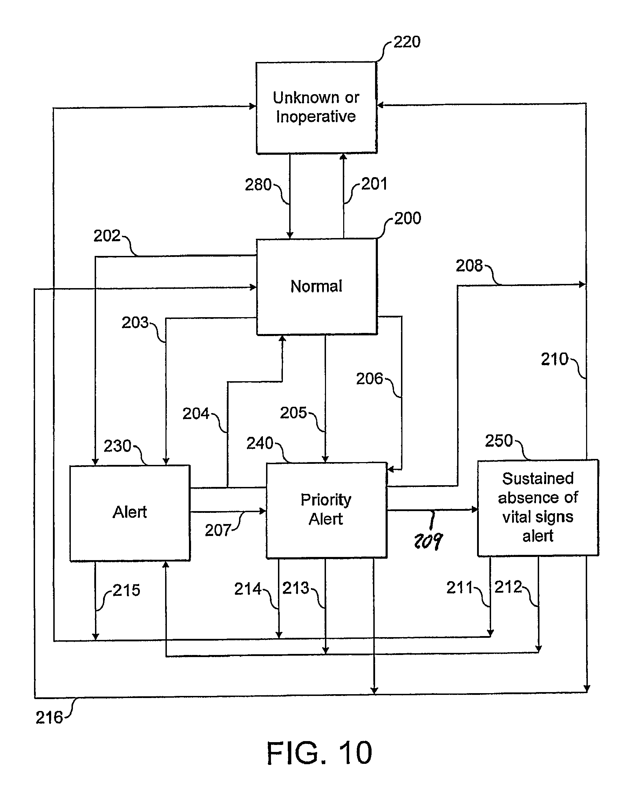

With reference to Figure 10, the cardio respiratory enumeration is computed according to the states and transitions shown. On application of power to the sensor, it will start at the UNKNOWN state 220 until it has completed its self-checks to determine the unit is working correctly and connected to a body. It will then transit into the NORMAL state 200, via 280. In the NORMAL state 200 it will monitor:
- a user's heart and respiration rate per minute (HR and BR) ;
- short-term near-instantaneous heart rate (HRst);
- short-term near-instantaneous breathing effort rate (BRst);
- long-term average heart rate over a number of different time windows (HRlt);
- the rate of change of heart rate over a time window; and,
- optionally, a user's blood oxygen level (Sp02).

The device will then compare these levels against a series of configurable thresholds and values, for example, as shown in the following table and if necessary it will determine a transition to an alert state.

| **Parameter** | **Description** | | **Range** |
|---|---|---|---|
| Heart Rate High Threshold | Upper limit for average heart rate per minute | | 0 - No Limit 1-255 beats per min (bpm) |
| Heart Rate Low Threshold | Lower limit for average heart rate per minute | | 0 - No Limit 1-255 bpm |
| Breathing Rate High Threshold | Upper respiration limit for average respiration rate per minute | | 0 - No Limit 1-255 bpm |
| Breathing Rate Low Threshold | Lower respiration limit for average respiration rate per minute | | 0 - No Limit 1-255 bpm |
| Short-Term Heart Rate (HRst)) TimeWindow | Time period over which a short term average heart rate is measured in order to provide an early indication of failure to detect any heart beats | | 0 - None 1-255 seconds |
| Short Term Breathing Rate (BRst) TimeWindow | Time period over which a short term average breathing rate is measured in order to provide an early indication of failure to detect respiration effort | | 0 - None 1-255 seconds |
| Sp02 Min Threshold | Lower limit of adequate blood oxygenation | | 0 - 100% |
| Long Term Heart Rate (HRlt)Threshold (s) | Upper limit for an average heart rate or rates measured over several different time window | | 0 - No Limit 1-255 bpm |
| Heart Rate Max Rate Threshold | Maximum change in heart rate without ambulation which may occur over time threshold 6 | | 0 - No Limit 1-255 bpm |
| Time Threshold 1 | Time required for an out of threshold rate to exist before an indication is raised | | 0 - Infinite 1 - 255 minutes |
| Time Threshold 2 | Time period when HR(st) = 0 before an indication is raised | | 0-255 seconds |
| Time Threshold 3 | Time period that BR(st) = 0 before an indication is raised | | 0-255 seconds |
| Time Threshold 4 | Time period to indicate sustained absence of vital signs. | | 0-Infinite 1- 255 minutes |
| Time Threshold 5 | Time period when HR = 0 and BR = 0 after which we indicate the high alert (red) state | | 0 - Infinite 1 - 255 minutes |
| Time Threshold 6 | Time period over which we measure if we exceed HR Max Threshold Change and an exception condition is raised | | 0 - Infinite 1 - 255 seconds |
| Time Threshold 7 | Time period that Temp > 39 or PSI is > PSIMax before an indication is raised | | 0-255mins |
| Time Threshold 8 | Time Period that we will wait for a neurological stimulation test response | | 0-255mins |
| Temp Hi Threshold | Surface temperature measurement (chest) upper limit for safe temperature regulation | | 0-45 deg C |
| PSI Max | Modified physiological strain index incorporating surface temperature and heart rate measures | | 0-10 |
| | MPSI | Heat Strain | |
| | 0 | | |
| | 1 | No/little | |
| | 2 | | |
| | 3 | Low | |
| | 4 | | |
| | 5 | Moderate | |
| | 6 | | |
| | 7 | High | |
| | 8 | | |
| | 9 | Very high | |
| | 10 | | |

If a users condition recovers back to within the boundaries defined in the sensor configuration, the welfare indication will return, via 204, to NORMAL 200. Those skilled in the art can see that the separation of certain combinations of physiology offers higher alert priority 205, 206, 207 to more immediately serious vitals signs states. Additionally, the detection of a condition known as ventricular fibrillation 206 is specifically identified for the same reasoning. In the alert states a neurological response test is automatically triggered and if the result is positive the indication transitions 216, 217, 218 to NORMAL 200. If the user indicates the need for assistance in his response the indication will remain or move to the ALERT 230 state, via 212,213. In the PRIORITY ALERT state 240, if the user condition does not recover by a time threshold then the indication will move to a SUSTAINED ABSENCE OF VITAL SIGNS 250, via 209. In the PRIORITY ALERT 240 or SUSTAINED ABSENCE OF VITAL SIGNS 250 state the detection of ambulation will cause the indication to transition to UNKNOWN 220, via 208,210, as this is inconsistent with the physiology being recorded.

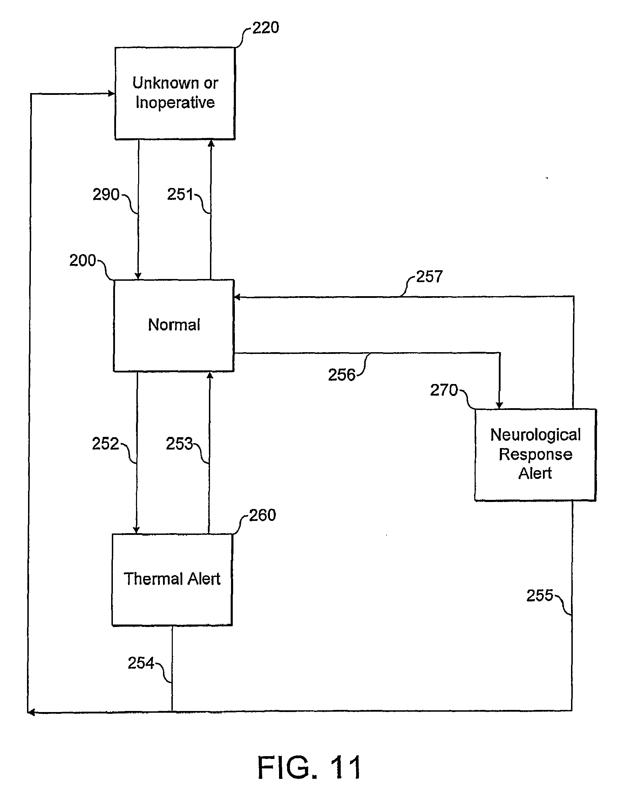

Referring to Figure 11, the secondary welfare indication is provided alongside the cardio respiratory welfare indication. The indication provides two alerts THERMAL ALERT 260 and NEUROLOGICAL RESPONSE ALERT 270. If a thermal exception is detected either due to the physiological index exceeding the configured value in the sensor or the skin temperature exceeding the maximum skin temperature, for a defined time period, then the indication will transition to this state 260, via 252. If this exception clears, then the indication will return to NORMAL 200, via 253. If the indication is in the NORMAL state 200 and a high gravitational shock to the body has been detected, a neurological test will be triggered and if no response is received within a defined time period and no ambulation is also detected then the indication will move to the NEUROLOGICAL RESPONSE ALERT state 270, via 256. The state may be cleared if subsequent ambulation is detected, or the user responds to a repeated neurological stimulation test, and the state is returned to NORMAL 200, via 257.

In all states, if the sensor detects a hardware failure which means its operation cannot be considered reliable, or the overall confidences in the cardio respiratory measures is reduced beyond a point where they may be inoperative, then indication will change state via transitions 215,214,211,201,254,255 to UNKNOWN 220.

## Claims

1. A monitoring device wearable by a person to be monitored, comprising:
one or more sensing means for sensing cardio, respiratory, physiological and/or other information from the person;
processing means for analysing the sensed information;
memory means for storing the sensed and/or analysed information; and
communication means for transmitting at least a portion of the analysed information, wherein:
at least one waveform acquired from the sensed cardio, respiratory, physiological and/or other information is digitised in real-time;
analysis of the sensed and/or digitised information is performed in real-time and a welfare indication of the person computed in real-time; and
the computed welfare indication of the person is transmitted by the communication means and/or stored in the memory means.

2. A monitoring device as claimed in claim 1, wherein the communication means is capable of transmitting sensed information.

3. A monitoring device as claimed in claim 1 or claim 2, wherein the communication means is capable of transmitting digitised information.

4. A monitoring device as claimed in any preceding claim, capable of transmitting the welfare indication, the sensed information and/or the digitised information in real-time.

5. A monitoring device as claimed in any preceding claim, capable of transmitting the welfare indication every 0.0001 to 60 seconds, more preferably every 5 to 30 seconds, and ideally every 15 seconds.

6. A monitoring device as claimed in any preceding claim, wherein the communication means is part of a radio, satellite and/or other communications network.

7. A monitoring device as claimed in any preceding claim, wherein all waveforms acquired from the sensed cardio, respiratory, physiological and/or other information are digitised in real-time.

8. A monitoring device as claimed in any preceding claim, wherein all sensed information is digitised in real-time.

9. A monitoring device as claimed in any preceding claim, wherein the sensing means comprises one or more skin electrodes.

10. A monitoring device as claimed in claim 9, wherein the sensing means comprises one or more electrodes and associated electronics circuitry.

11. A monitoring device as claimed in any preceding claim, wherein the one or more sensing means comprises at least two sensing means.

12. A monitoring device as claimed in any preceding claim, wherein at least part of the memory is a buffer-type memory.

13. A monitoring device as claimed in any preceding claim, wherein the processor is capable of processing at least two forms of information selected from cardio, respiratory, physiological and/or other information, to derive data relating to a welfare indication of a wearer.

14. A monitoring device wearable by a person to be monitored, comprising:
a detachable anatomically-shaped sensor electronics module comprising processing means, memory means and communications means; and
a connector, harness and/or other support wearable by a person, capable of attaching, or holding in sensory/sensing proximity, the sensor electronics module to a person, and comprising one or more sensing means, wherein the monitoring device:
senses cardio, respiratory, physiological and/or other information from a person; and
performs real-time analysis of the sensed information and computes a real-time welfare indication of the person for onwards transmission / communication.

15. A monitoring system for monitoring of one or more persons comprising:
a monitoring device as claimed in any one of claims 1 to 14, wearable by the or each person being monitored; and
one or more monitoring stations, wherein:
the or each monitoring device is in communication with the one or more monitoring stations; and
the one or more monitoring stations receive and monitor the computed welfare indication from the or each monitoring device to assess the wellbeing of each person being monitored.
